# EUROPEAN PATENT APPLICATION

(11) **EP 3 505 160 A1**
(43) Date of publication of application: **03.07.2019**
(21) Application number: 17020596.7
(22) Date of filing: 31.12.2017
(51) Int. Cl.: A61K 9/20, A61K 31/5377

(54) **PREPARATION OF A SOLID PHARMACEUTICAL COMPOSITION COMPRISING RIVAROXABAN AND PRODUCTION THEREOF**

(71) Applicant: Abdi Ibrahim Ilac Sanayi ve Ticaret A.S., 34500 Esenyurt/Istanbul (TR)
(72) Inventor: Bugra Dogru, Melik, Esenyurt- Istanbul (TR); Dude Kumar, Udaya, Esenyurt- Istanbul (TR)

(57) **Abstract**

The present invention relates to a prodution of rivaroxaban tablets by direct compression and preparation thereof.

## Description

### Technical Field of Invention

The present invention relates to a prodution of rivaroxaban tablets by direct compression and preparation thereof.

### Background of the Invention

Rivaroxaban is a selective and strong anti-coagulation factor Xa, disclosed in WO 01/47919 patent. Chemical name is (S)-5-chlor-N-{2-oxo-3-[4-(3-oxomorpholin-4-yl)phenyl]-1,3-oxazoldin-5-yl methyl]thiophen -2-carbamid and its chemical structure is shown below.

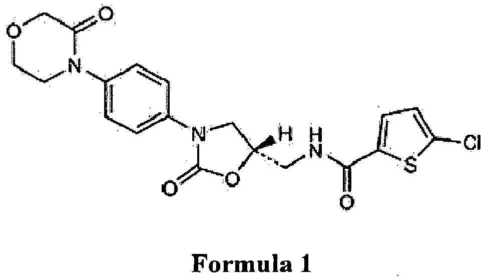

Rivaroxaban film coated tablet formulations are available in market at 2.5 mg, 10 mg, 15 mg ve 20 mg dosages with Xarelto ® commercial name. Xarelto ® is used in the prevention and treatment of atherothrombotic disorders such as heart attack, chest angina, angioplasty, or post-bypass reocclusion and restenosis, transient ischemic attack, stroke and systemic embolism, pulmonary embolism, deep vein thrombosis and venous thromboembolism.

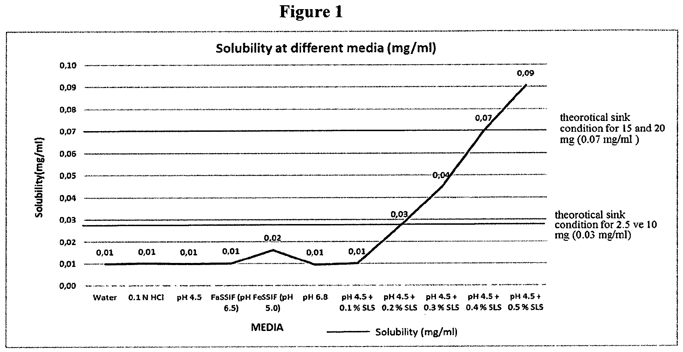

Rivaroxaban has no pH range or solubility in water. According to the Biopharmaceutical Classification System, it is a Class II drug which has low solubility and high permeability. Therefore, dissolution and absorption of the drug is important. (Figure 1)

In prior art, EP1689370 B1 patent discloses the production method of solid oral pharmaceutical composition by wet granulation. The use of fluid bed granulation as wet granulation method and the use of surfactant preferably sodium lauryl sulfate(SLS) are disclosed. It is also mentioned that the tablets produced with wet granulation in the examples provide increased bioavailability by 35 % compared to tablets produced by direct compression.

WO 2016/166733 A1 discloses granulation of rivaroxaban and pharmaceutically acceptable excipients by mixing with non-aqueous solvent.

WO 2014/191446 A1 dicloses the milling of rivaroxaban and hydrophilic binder mixture. Preferably, sodium lauryl sulfate(SLS) is added to mixture a as surfactant.

WO 2011/042156 A1 discloses the granulation of rivaroxaban, a hydrophilic matrix former and a disintegrant by a melt-extrusion process at a temperature that the crystalline form of (form I) of rivaroxaban does not change. In addition, mixture can contain surfactant preferably sodium lauryl sulfate.

WO 2010/146179 A2 discloses the hot melt granulation of rivaroxaban or a pharmaceutically acceptable salt or solvate thereof and at least one pharmaceutically acceptable excipient.

WO 2010/003641 A1 discloses the preparation of mixture comprising rivaroxaban, solvent and pseudo-emulsifier by dry granulation. This process comprises the production steps of mixing of active ingredient and excipients, compressing tablets and granulation of tablets. Surfactant preferably sodium lauryl sulfate(SLS) is also used as excipient.

In conclusion, various techniques have been used in the state of art to increase the solubility of rivaroxaban. Wet granulation, hot melt granulation, spray granulation processes are used to increase the solubility of active substance with low solubility in a way that they increases the interaction between active ingredient and surfactant. In addition in EP1689370 B1, it is disclosed that rivaroxaban is in the hydrophilized form to obtain high bioavailability.

However, inventors surprisingly produced a solid oral pharmaceutical formulation comprising rivaroxaban with high bioavailability by direct compression.

### Description of the Invention

The objective of the present invention is to produce rivaroxaban tablets by direct compression and preparation thereof.

In the present invention, high bioavailability was achieved by direct compression of tablets comprising rivaroxaban without any granulation process. In addition, there is no milling process of rivaroxaban with a hydrophilic excipient to convert rivaroxaban in a hydrophilized form to increase bioavailability. In particular, inventors obtained products with high bioavailability by using poloxamer as surfactant.

### Detailed Description of the Invention

The present invention relates to a solid oral pharmaceutical composition comprising rivaroxaban wherein the composition comprises at least one surfactant and at least one pharmaceutically acceptable excipient.

The term "Rivaroxaban" refers to rivaroxaban, pharmaceutically acceptable salt, hydrates, esters, derivatives or solvates of thereof. Rivaroxaban can be in crystalline and amorphous form, preferably it is in crystalline form. More preferably, in present invention, modification form I, which is physically and chemically more stable during production and shelf life, is used.

The term pharmaceutical composition is for oral use. The pharmaceutical composition to be used orally can be in the dosage forms such as tablets (such as tablets, film-coated tablets, chewable tablets, effervescent tablets, layered tablets, soluble tablets, sublingual tablets, orally disintegrating tablet), capsules (such as soft capsules and microcapsules), granules, grain, powder, pills or mixtures thereof. The pharmaceutical composition of the present invention is preferably in tablet form and may optionally be film coated.

In another emboidment of the present invention is to provide a tablet comprising rivaroxaban with high bioavailability.

It is found that pharmaceutical compositions comprising rivaroxaban with high bioavailability can be obtained using direct compression without any need to granulation step. In addition, there is no need for milling process of rivaroxaban with hydrophilic excipient to obtain a hydrophilized form of rivaroxaban to improve bioavailability. It is found that if a surfactant (eg. Poloxamer) is used in a sufficient ratio, sufficiently high bioavailability is obtained. As a result of trials done by inventors, the total weight of the pharmaceutical composition should comprise in the range of 0.01-2%, preferably 1.8%, more preferably 1.6% surfactant by weight.

In one emboidment of the present invention, a production method of tablets comprising rivaroxaban, comprises the following steps:
(i) preparing a mixture of rivaroxaban, at least one surfactant and at least one pharmaceutically acceptable excipient,
(ii) direct compression of tablets by the mixture obtained in step (i)

"Powder mixture" refers to mixing of each component individually not obtaining by granulation method such as wet granulation and dry granulation.

In a preferred emboidment of the present invention, rivaroxaban is used in micronized form. Mean particle size distribution (d₅₀) is preferably 10 µm or less, more preferably is in the range of 1-8 µm. The value of d₉₀ is preferably 20 µm or less, more preferably is in the range of 1-15 µm.

The term "d₅₀ particle size" used herein to average particle size of the particles and refers to average particle diameter by volume. "d₉₀ particle size" refers to a particle size of 90% of particles by volume. d₅₀ and d₉₀ values can be measured by using a known measuring device such as the instrument measuring particle distribution by laser diffraction (e.g. Malvern MasterSizer). The measurements can be done by wet or dry method in laser diffraction instrument. Preferebly dry method is used in the present invention.

The term "surfactant" used herein refers to chemical compounds that affect (often reduce) surface tension when dissolved in water or an aqueous solution. Surfactants also affect the interfacial tension between the two liquids. Surfactants also affect the interfacial tension between two liquids

Surfactants which can be used in this composition can include at least one of sodium lauryl sulfate (SLS), poloxamer, docusate sodium, polysorbates, or mixtures thereof and is not limited thereto. Preferably it is poloxamer.

In another embodiment of this invention, any solid poloxamer can be used as the surfactant for the preparation of the pharmaceutical composition. Poloxamers in the market are waxy alcohols having a melting point between 45-60 ° C. Preferably, poloxamer 188 having a melting point in the range of 52-57 ° C is used.

The poloxamer is a non-toxic, non-irritating pharmaceutical excipient and can be used in oral formulations up to 10% (w/w) without causing gastric irritation. Preferably, the tablets of the present invention comprises poloxamer from 0.5 to 5.0% (w / w), preferably from 0.8 to 4.0% (w/w), more preferably from 1.4 to 3.6% (w/w) and most preferably 1.6% of the total weight of the tablet or the total weight of the core tablet in case of film coated tablet.

In the present invention, the ratio of rivaroxaban to surfactant is in the range of 10:1 to 1:1, preferably 7:1 to 1.3:1.

In a preferred emboidment of the invention, pharmaceutically acceptable excipients are used. Pharmaceutically acceptable excipients comprise at least one filler(diluent), at least one disintegrant, at least one binder, at least one lubricant, at least one glidant, at least one colorant, at least one sweetener and coating materials. Preferably, tablet, if coated core tablet comprises rivaroxaban, at least one surfactant, at least one binder, at least one filler (diluent), at least one disintegrant and at least one lubricant.

The term "binder" used herein refers to at least one of hydroxypropyl methyl cellulose, methyl cellulose, hydroxyethylcellulose, hydroxyethylmethylcellulose, hydroxypropyl cellulose, polyvinylpyrrolidone (povidone), polyvinyl alcohol, copovidone and pregelatinized starch or mixtures thereof and is not limited thereto. Preferably, it is hydroxypropyl methyl cellulose.

The term "filler (diluent)" used herein refers to at least one of lactose monohydrate, anhydrate lactose, microcrystalline cellulose, calcium hydrogene phosphate, calcium phosphate and mannitol or mixtures thereof and is not limited thereto. Preferably, it is the mixture of lactose and microcrystalline cellulose.

The term " disintegrant" used herein refers to at least one of croscarmellose sodium, sodium starch glycolate, crospovidone, microcrystalline cellulose, low substituted hydroxypropyl cellulose, povidone, starch, pregelatinized starch, sodium or calcium alginate, methyl cellulose, agar, alginic acid or mixtures thereof and is not limited thereto. Preferably, it is croscarmellose sodium.

The term "lubricant" used herein refers to at least one of magnesium stearatei sodium stearyl fumarate, hydrogenated vegetable oil, talc or mixtures thereof and is not limited thereto. Preferably, it is magnesium stearate.

It is known in the art that wet granulation has an advantage to obtain content uniformity of the active ingredient. In order to ensure content uniformity in direct compression method; the powder mixture subjected to direct compression in the present invention;
a) rivaroxaban, at least one surfactant and a portion of at least one pharmaceutically acceptable excipient(s) are mixed and sieved,
b) optionally obtained powder mixture is sieved once or more,
c) rest of the pharmaceuticaly acceptable excipient(s) is added to powder mixture and optionally sieved,
d) Lubricant is added to powder mixture and optionally sieved,
e) Powder mixture is brought to desired dosage form.

Preferably, rivaroxaban, at least one surfactant, at least one binder and a portion of at least one filler(diluent) is sieved. In a preferred embodiment of the invention, the mixture obtained in step (a) is mixed with at least one disintegrant, at least one lubricant and the rest of at least one filler (diluent) to obtain powder mixture. In addition to this, it is sieved optionally before the step (d) namely addition of lubricant. Mixtures obtained in the production of this composition and/or pharmaceutically acceptable excipients are sieved from 0.1 mm to 1.0 mm, preferably 0.63 mm screen.

In one embodiment of the invention, the preparation of the rivaroxaban tablet by direct compression comprises the steps of:
a) mixing rivaroxaban, at least one surfactant and at least one binder and a portion of at least one filler(diluent) and sieving,
b) optionally sieving the obtained mixture once or more,
c) adding rest of at least one filler(diluent) and at least one disintegrant to obtained mixture and sieving optionally,
d) adding lubricant to mixture and mixing,
e) compressing the tablet.

In the present invention, excipients having particle size of d₉₀<250 µm are selected.

The term "direct compression" used herein refers to compression of tablets from powder mixture without any addition and /or any process.

In a preferred embodiment of the present invention, the preparation of the rivaroxaban tablet by direct compression comprises the steps of:
a) mixing rivaroxaban, poloxamer and hydroxypropyl methylcellulose and a portion of lactose and sieving,
b) optionally sieving the obtained mixture once or more,
c) adding rest of lactose, microcrystalline cellulose and croscarmellose sodium to obtained mixture and sieving optionally,
d) adding magnesium stearate to mixture and mixing and sieving optionally,
e) compressing the tablet with suitable punches.

In the present invention, the composition comprises 1.6 % poloxamer by weight by weight of the total tablet weight and the mean particle size(d₅₀) of the rivaroxaban is 10 µm or less, preferably in the range of 1 and 8 µm, the value of d₉₀ is preferably 20 µm or less, more preferably in the range of 1 and 15 µm.

The present invention is further defined by reference to the following examples. The examples are not intended to limit the scope of the present invention, they should be considered in the light of the description detailed above.

### Example 1:

| **Ingredients** | **mg/tablet** | | |
|---|---|---|---|
| | **Example** 1 | **Example 2** | **Example 3** |
| *Rivaroxaban (modification I)* | 20 | 20 | 20 |
| *Microcrystalline Cellulose* | 87 | 87 | 83.8 |
| *Lactose* | 76.5 | 74.9 | 74.9 |
| *Croscarmellose sodium* | 7.64 | 7.64 | 7.64 |
| *Hydroxypropylmethyl cellulose* | 6.24 | 6.24 | 6.24 |
| *Poloxamer 188* | 1.6 | 3.2 | 6.4 |
| *Magnesium stearate* | 1.02 | 1.02 | 1.02 |
| ***Core tablet weight*** | **200** | **200** | **200** |

### Production method:

1. Sieve rivaroxaban, poloxamer, HPMC and a portion of lactose two times.
2. Add microcrystaline cellulose croscarmellose sodium and rest of lactose to the mixture obtained in step (1).
3. Add magnesium stearate and mix.
4. Compress tablet with suitable punches.
5. Coat tablets with 10% (w/w) coating solution.

Dissolution analysis were performed in pH 4.5 acetate buffer 0.4% SLS medium at 75 rpm. Obtained dissolution profiles of the examples were compared to reference product Xarelto® 20 mg Film coated Tablet.

**TABLE 1- Comparative dissolution results of samples and reference product**

| **Time** | **% Çözünme Hizi** | | | |
|---|---|---|---|---|
| **(minute)** | **Xarelto®** | **Example 1** | **Example 2** | **Example 3** |
| 5 | 54.8 | 60.2 | 69.7 | 70.1 |
| 10 | 88.6 | 70.8 | 80.6 | 81.0 |
| 15 | 94.7 | 76.0 | 86.2 | 85.8 |
| 20 | 97.1 | 79.4 | 88.3 | 88.2 |
| 30 | 97.7 | 83.9 | 91.4 | 91.4 |
| 45 | 99.4 | 87.4 | 93.5 | 93.7 |
| 60 | 99.6 | 89.2 | 94.9 | 95.2 |

When analyzing examples and Table-1, the ratio of poloxamer is increased to 0.8 % (w/w), 1.6 % (w/w) and 3.2 % (w/w) in example 1, example 2 and example 3 respectively, an increase in the dissolution result of rivaroxaban was observed. However, no significant difference in dissolution results of example-2 and example-3 was observed, therefore the optimum concentration of poloxamer is determined as 1.6%.

## Claims

1. A production process of a tablet comprising rivaroxaban wherein it comprises the following steps of:
i) preparing a powder mixture comprising rivaroxaban, surfactant and pharmaceutically acceptable excipients,
ii) subjecting the powder mixture obtained in step (i) to compress tablet by direct compression method.

2. According to claim 1, pharmaceutically acceptable excipients are binders, fillers (diluent), disintegrants, lubricants.

3. According to claim 1, the ratio of rivaroxaban to surfactant is in the range of 10:1 to 1:1, preferably 7:1 to 1.3:1.

4. A process according to claim 1, wherein powder mixture comprises the following steps of:
a) sieving rivaroxaban, surfactant and a portion of at least on pharmaceutically excipient,
b) sieving the mixture optionally,
c) repeat the step b) optionally once or more,
d) mixing a mixture obtained in step a), b), c) with rest of pharmaceuticall acceptable excipient to obtain powder mixture

5. According to claim 1 or claim 2, particle size distribution of excipients is d₉₀ < 250 µm.

6. A process according to claim 1, wherein powder mixture comprising rivaroxaban, surfactant and pharmaceutically acceptable excipients is compressed to tablet by direct compression.

7. According to claim 1, surfactant is selected from at least one of sodium lauryl sulphate(SLS), poloxamer, docusate sodium, polysorbates and mixtures thereof, preferably it is poloxamer

8. According to claim 1, tablets are coated optionally.
